# EUROPEAN PATENT SPECIFICATION

(11) **EP 3 141 897 B1**
(45) Date of publication and mention of the grant of the patent: **12.02.2020**
(21) Application number: 16188167.7
(22) Date of filing: 09.09.2016
(51) Int. Cl.: G01N 33/28, G01N 24/08

(54) **PREDICTION METHOD OF CHEMICAL-PHYSICAL PROPERTIES OF A PETROLEUM DISTILLATION FRACTION**
VERFAHREN ZUR VORHERSAGE CHEMISCH-PHYSIKALISCHER EIGENSCHAFTEN VON ERDÖLDESTILLATIONSFRAKTION
PROCÉDÉ DE PRÉDICTION DE PROPRIÉTÉS PHYSICO-CHIMIQUES D'UNE FRACTION DE DISTILLATION DE PÉTROLE

(30) Priority: 09.09.2015 IT UB20153510
(43) Date of publication of application: 15.03.2017
(73) Proprietor: Saras Ricerche e Tecnologie S.p.A., 09032 Assemini (CA) (IT); Universita' Degli Studi Di Cagliari, 40100 Cagliari (IT)
(72) Inventor: PULIGHEDDU, Sonia, 09032 ASSEMINI CA (IT); SASSU, Lorenzo, 09032 ASSEMINI CA (IT); MASILI, Alice, 40100 CAGLIARI CA (IT)
(74) Representative: Santi, Filippo

(56) References cited:
- EP-A1- 0 859 952
- WO-A1-2008/135411
- WO-A1-2013/102916
- US-A1- 2015 106 027

## Description

The present invention relate to a prediction method of chemical-physical properties of a fraction of distillation of a petroleum (oil).

More in detail, the invention relates to a prediction method of the properties of a fraction of distillation of a oil, which can be implemented without the need to carry out the actual distillation, and still more in detail a prediction method of the properties of the residue of a oil, wherein by residue it is intended a heavy fraction of the dstillation of oil.

At present, to meet the reduction in margins of refineries, refiners have to respond to a growing demand for distillates with increasingly stringent requirements and to the need to refine cheaper and cheaper oils of lower quality. This trend, and the need to even better exploit the less valuable fractions of distillation, has shifted the focus to the processes of conversion of residues, which may constitute up to 60% by weight of oil. This requires a more conscious approach to refining, which passes through the optimization of the production, the improvement of the selection of the oils and of the mixtures to be processed, and which can only be obtained through a detailed knowledge of the composition and the quality of the oil and of the semi-finished products.

Generally, the detailed characterisation of oil and its fractions involves the distillation and the execution of a series of analyses on fractions, with reference methods such as for example UOP, IP, ASTM. Only by referring to the latter organization, a list of the analysis by way of example includes: ASTM D2892-11a, "Distillation of Crud" Oil (15-Theoretical Plate Column)"; ASTM D1160-12, "Standard Test Method for Distillation of Oil Products at Reduced Pressure"; ASTM D1298-12b, "Density, Relative Density (Specific Gravity), or API Gravity of Crude Oil and Liquid Oil Products by Hydrometer Method"; ASTM D2622-10, "Sulfur in Oil Products by Wavelength Dispersive X-ray Fluorescence Spectrometry"; ASTM D664-11a, "Acid Number of Oil Products by Potentiometric Titration"; ASTM D445-12, "Standard Test Method for Kinematic Viscosity of Transparent and Opaque Liquids (and Calculation of Dynamic Viscosity)"; ASTM D4530-11, "Standard Test Method for Determination of Carbon Residue (Micro Method)"; ASTM D6560-12, "Standard Test Method for Determination of Asphaltenes (Heptane Insolubles) in Crude Oil and Oil Products1,2"; ASTM D5291-10, "Standard Test Methods for Instrumental Determination of Carbon, Hydrogen, and Nitrogen in Oil Products and Lubricants".

This procedure, named "Crude assay", takes about 2-3 weeks, and involves the use of large quantities of samples, consumables, as well as specialized personnel. In particular, the characterisation of the atmospheric residue (AR) and vacuum residue (VR) from oil always requires, first of all, in addition to the execution of the atmospheric distillation (TBP) to obtain the atmospheric residue, also the additional fractionation of the latter with the vacuum distillation (Potstil), to obtain the vacuum gas oil and the vacuum residues. All fractions must then be analytically characterised. The residue analysis procedure is therefore particularly long and complex.

In this context, the development of fast and accurate testing methods to replace traditional laboratory methods has become of paramount importance in many areas of refining, ranging from planning to monitoring and process control. Various techniques using NMR spectroscopy, UV, Visibile, Raman, near infrared (NIR) and mid infrared (MIR) can provide, with a different level of detail, characterisation data quickly and at low cost.

Given the very complex nature of the oil matrixs, to exploit the content of information in the spectra, usually, use is made of chemometric analysis techniques, which correlate the spectral data with the chemical-physical properties, for example through the application of a partial least squares (PLS) regression model.

Scientific literature is very limited. Chung [Chung H., and Ku M., Applied Spectroscopy 2000, 54] compared the use of NIR, IR and Raman spectroscopy applied to atmospheric residue (AR) to estimate the API degree, Hongfu [Hongfu Y., Xiaoli C., Haoran L., and Yupeng X., Fuel 2006, 85] and Satya [Satya S., Roehner R. M., Deo M. D., and Hanson F. V., Energy & Fuels 2007, 21] developed PLS models based on NIR and ATR-FTIR spectra of residues, to determine a number of parameters including the amount of Saturated, Aromatics, Resins and Asphaltenes (SARA), carbon residue (MCRT), C/H, C, H and N. Finally, Muller [Müller A.L.H., Picoloto R.S., de Azevedo Mello P., Ferrão M.F., dos Santos M.d.F.P., Guimarães R.C.L., Müller E.I., and Flores E.M.M., Spectrochimica Acta 2012, Part A 89*]* determined the total content of sulfur in atmospheric and vacuum residues, by means of ATR-FTIR spectroscopy.

Nielsen first [Nielsen K.E., Dittmer J., Malmendal A., and Nielsen N.C., Energy & Fuels 2008, 22] used NMR spectroscopy in conjunction with the multivaried regression (PLS) to estimate carbon residue (MCRT), sulfur, water, density and calorific value of the residues, and then Molina [Molina V.D., Uribe U.N., and Murgich J., Energy & Fuels 2007, 21] used the same technique to determine the SARA content in vacuum residues.

The patent EP859952B1 describes a method for predicting the properties of oil fractions by exploiting the correlation between their IR spectrum and the properties of interest.

While all the above examples provide for the oil fractionation to obtain the residue, the first document which describes the possibility of being able to estimate the properties of the residue directly from the regression models developed starting from the absorbances of the oil IR signals, is the patent EP304232B1, in the examples of application N. 17-19. However, this patent does not provide for regression models to include, in addition to the spectra, one or more descriptive physical properties of oil.

Subsequently, De Peinder [De Peinder P., Petrauskas D.D., Singelenberg F., Salvatori F., Visser T., Soulimani F., and Weckhuysen B.M., Applied Spectroscopy 2008, 62*;* De Peinder P., Visser T., Petrauskas D.D., Salvatori F., Soulimani F., Weckhuysen B.M., Vibrational Spectroscopy 2009, 51.] verified the possibility of using the IR spectra of oil to estimate the properties of the atmospheric residue as a function of the flash temperature. The related procedure is also subject of the European patent EP2142908B1 (originally published as WO2008/135411). The patent describes a method to predict the physical chemical properties of a residue that can be obtained from the distillation of crude oil (at certain parametric conditions) by applying a correlation-based predictive model that includes characteristic parameters of the distillation process. The method does not require correlations to include, descriptive physical chemical properties of oil.

The estimates obtained in the two cases mentioned above are smaller than those obtainable directly from the spectra of the residues and distant from reference values of the conventional analytical methods.

The international application WO2013/102916 describes a method for the prediction of the processability, quality and cost of oil and its distillates through correlations that can also use the physical chemical properties of oil, obtained through standard analytical methods. However, the method does not involve the use of spectroscopic data.

In the light of the above, it appears evident that the possibility to improve the predictions of properties of the residue, and more generally of a distillation fraction, directly from the oil would be extremely important to ensure that data can be used for better management, also economic management, of the refining processes.

In this context it is included the solution according to the present invention, which aims to provide a method to obtain the information contained in the NMR spectrum of an oil connected with the chemical-physical properties of the fractions of atmosferic and vacuum distillation, by exploiting the correlation that exists between the oil properties and the properties of its fractions, consequently significantly improving the capacity of prediction of the properties of industrial interest of distillation fractions.

Purpose of the present invention is therefore to provide a prediction method of chemical-physical properties of a fraction of a oil distillation which overcomes the limits of the prediction methods according to the prior art and to obtain the technical results previously described.

Another aim of the invention is that said prediction method can be implemented with substantially low costs.

Another object of the invention is to propose a prediction method of chemical-physical properties of a fraction of a oil distillation that is simple, safe and reliable.

It is therefore a specific object of the present invention a prediction method of chemical-physical properties of a fraction of atmospheric and/or vacuum distillation of a oil, as defined according to any of the claims 1-3.

Additional features of the invention are shown in the subsequent dependent claims.

The invention will be described below for illustrative, but not limitative, purposes, with particular reference to some illustrative examples and to the figures of the accompanying drawings, in which:

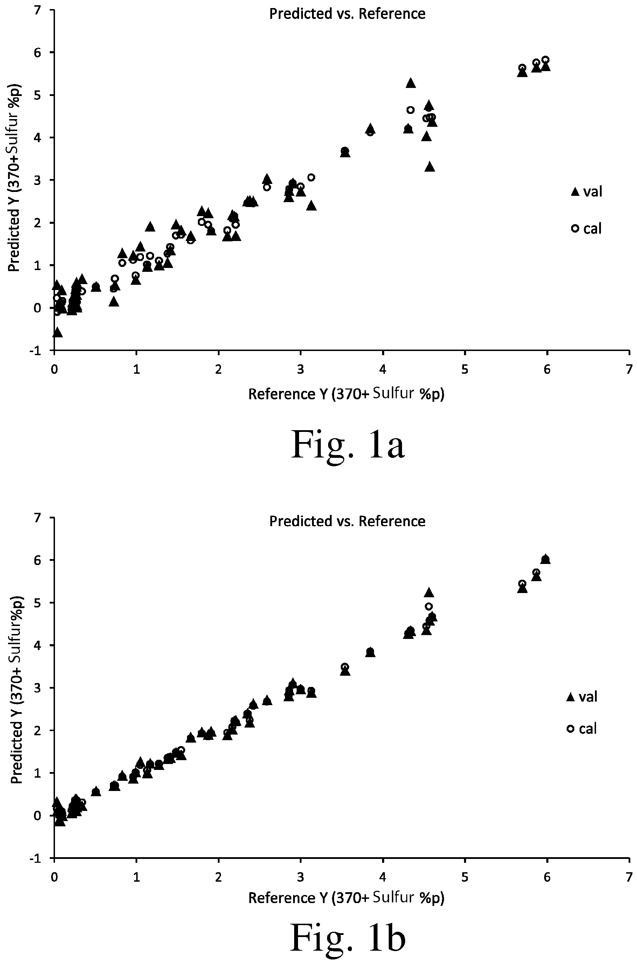

- Figures 1 and 1b show the measured values and the values calculated with the prediction model concerning sulfur of atmospheric residues, respectively from the matrix X of spectral data determined as described in examples 3 and 4 (Figure 1a) and from the matrix Z or expanded matrix (Figure 1b), formed by the X matrix of the ¹H NMR spectra of oils and the chemical-physical properties, as described also in the examples 3 and 4, in which the circles represent in the calibration samples (CAL), and the triangles are the samples of the validation set (CROSS-VAL),

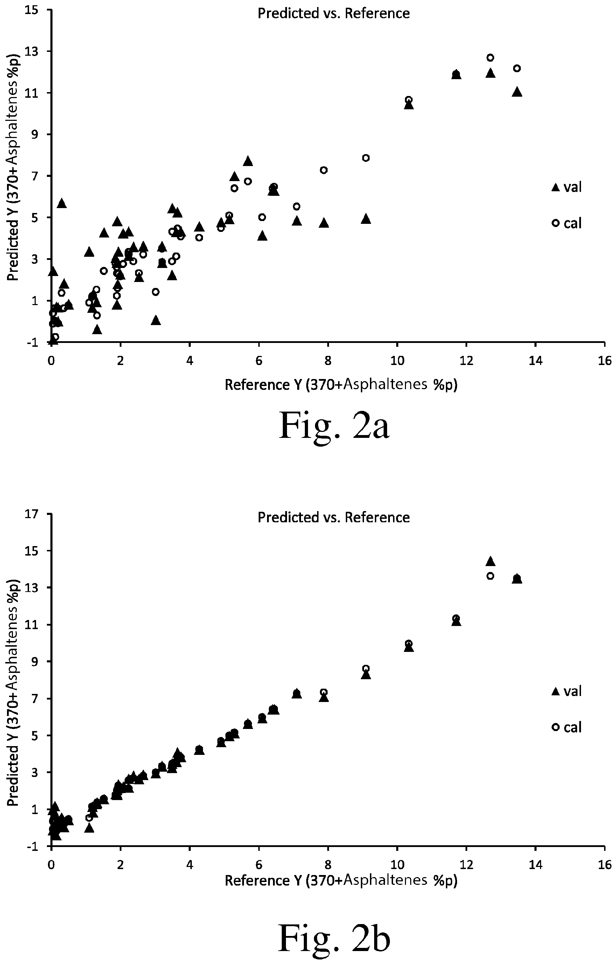

- Figures 2a and 2b show the measured values and the values calculated with the prediction model concerning asphaltenes of atmospheric residues, respectively from the matrix X (Figure 2a) and from the matrix Z (Figure 2b), in which the circles represent the calibration samples (CAL), and the triangles are the samples of the validation set (CROSS-VAL),

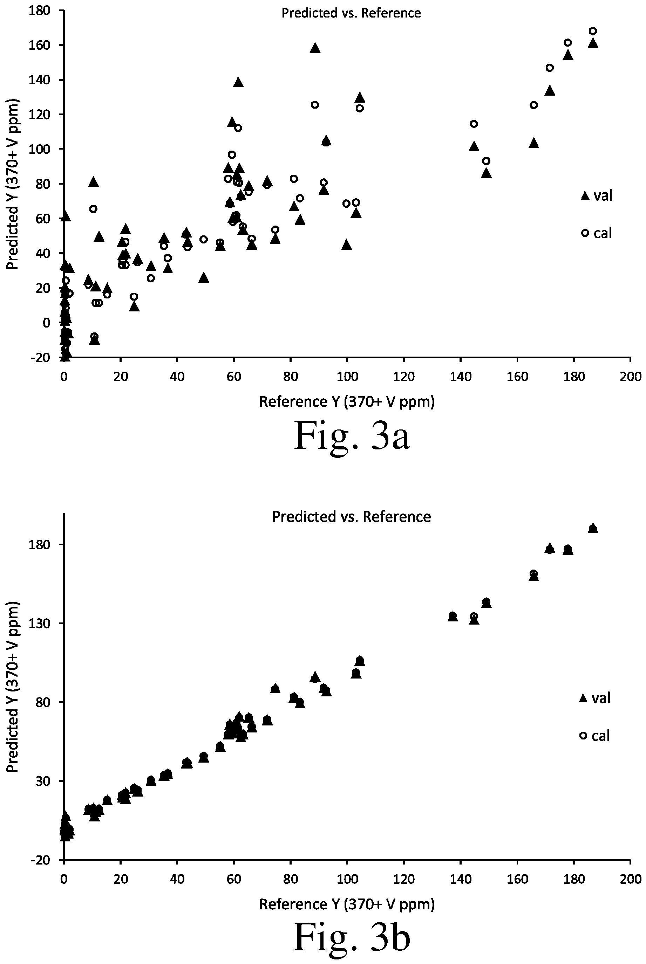

- Figures 3a and 3b show the measured values and the values calculated with the prediction model concerning the vanadium of atmospheric residues, respectively from the matrix X (Figure 3a) and from the matrix Z (Figure 3b), in which the circles represent in the calibration samples (CAL), and the triangles are the samples of the validation set (CROSS-VAL),

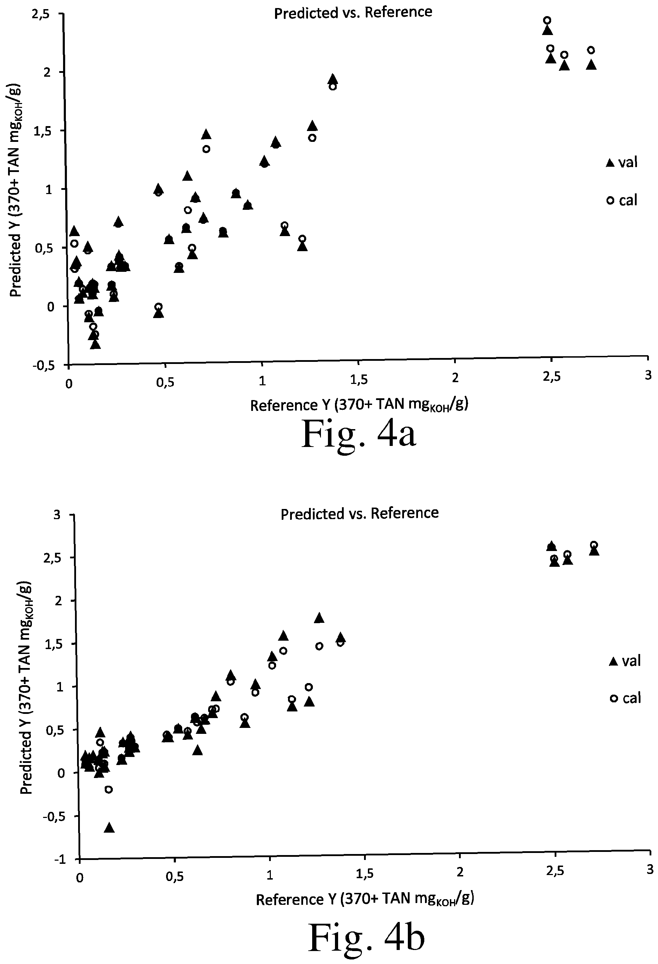

- Figures 4a and 4b show the measured values and the values calculated with the prediction model concerning acidity (expressed as total acid number, TAN) of atmospheric residues, respectively, from the matrix X (Figure 4a) and from the matrix Z (Figure 4b), in which the circles represent the calibration samples (CAL), and the triangles are the samples of the validation set (CROSS-VAL),

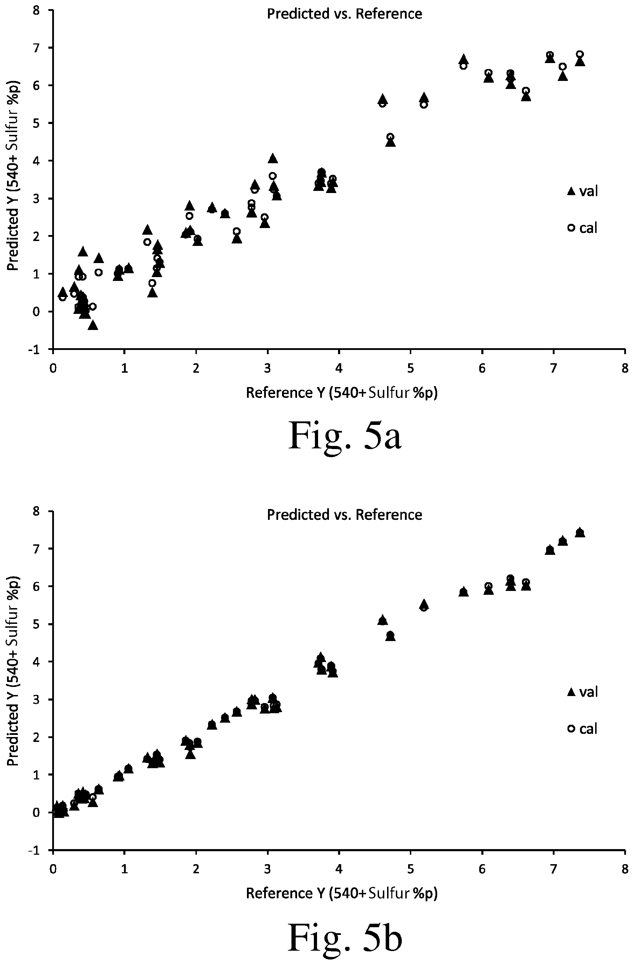

- Figures 5a and 5b show the measured values and the values calculated with the prediction model concerning sulfur of vacuum residues respectively from the matrix X (Figure 5a) and from the matrix Z (Figure 5b), in which the circles represent the calibration samples (CAL), and the triangles are the samples of the validation set (CROSS-VAL)

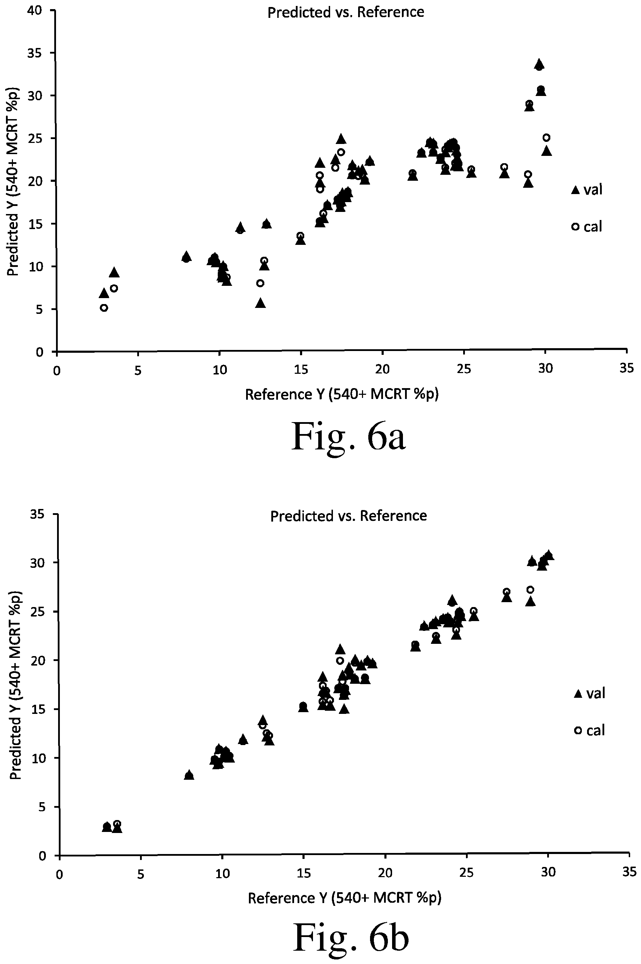

- Figures 6a and 6b show the measured values and the values calculated with the prediction model concerning the MCRT carbon residue of vacuum residues respectively from the matrix X (Figure 6a)and from the matrix Z (Figure 6b), in which the circles represent in the calibration samples (CAL), and the triangles are the samples of the validation set (CROSS-VAL),

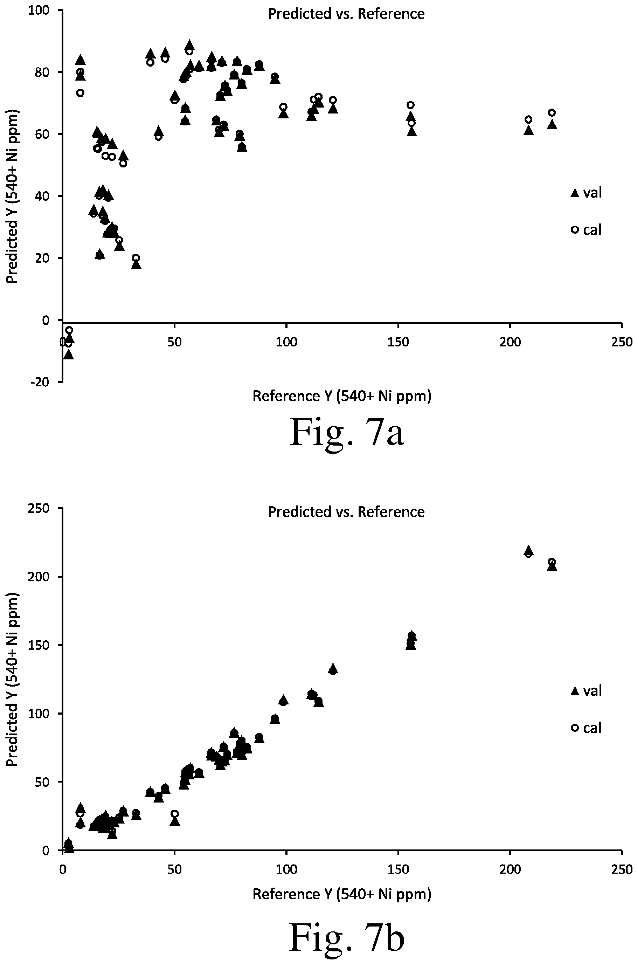

- Figures 7a and 7b show measured values and the values calculated with the prediction model concerning nickel of vacuum residues respectively from the matrix X (Figure 7a) and from the matrix Z (Figure 7b), in which the circles represent the calibration samples (CAL), while the triangles are the samples in the validation set (CROSS-VAL), and

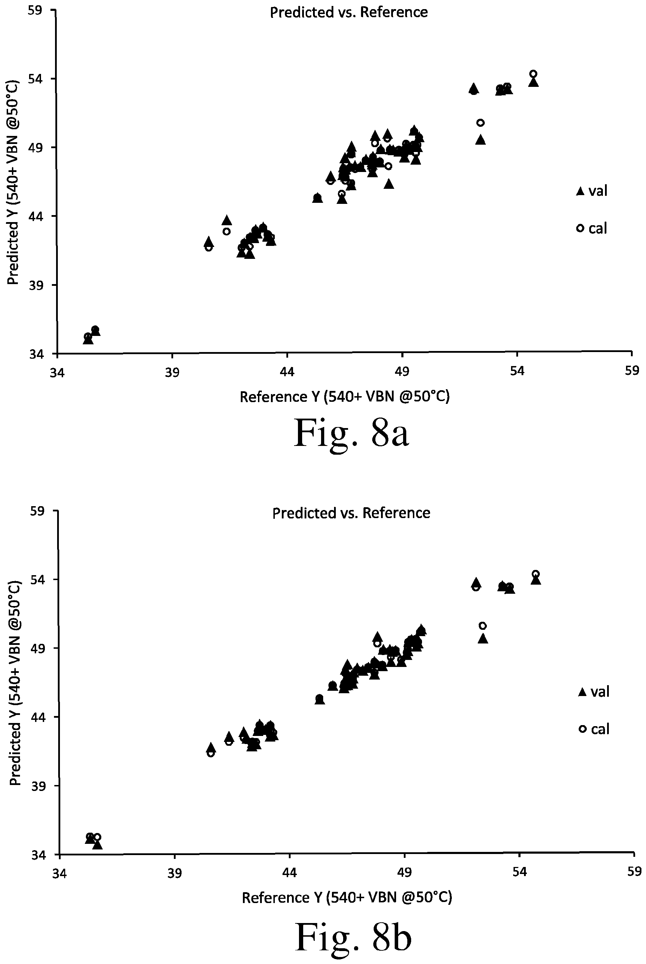

- Figures 8a and 8b show the measured values and the values calculated with the prediction model concerning the viscosity (Viscosity Blending Number, VBN) of vacuum residues respectively from the matrix X (Figure 8a) and from the matrix Z (Figure 8b), in which the circles represent the calibration samples (CAL), and the triangles are the samples of the validation set (CROSS-VAL).

The analytical method developed according to the present invention allows to accurately estimate the properties of a fraction of atmospheric distillation and a fraction of vacuum distillation, without carrying out the distillation, through the application of a partial least squares (PLS) regression model, starting from the spectrum ¹H NMR of the oil, which is concatenated with selected chemical-physical properties, respectively, of oil and of the fraction of atmospheric distillation.

By way of experimental verification, the prediction method of chemical-physical properties of a distillation fraction of oil according to the present invention was applied for the estimation of: sulfur content, total acidity (TAN), asphaltenes, carbon residue (MCRT), viscosity (Viscosity Blending Number, VBN) and metal content. These properties are important for the purposes of process monitoring and are particularly useful for the programming and management of the installations. For the development of the method 70 oil samples have been used from 18 different geographical areas with a wide range of chemical-physical properties. The models have been developed using a unique set for the calibration and validation, and the performance of the model in terms of mean square error, were assessed in a cross-validation. The results obtained show that the addition of information of a chemical physical nature to the spectral data is an effective method to improve the performance of chemometric models based on ¹H NMR, in the case in which the relation between the spectrum and the parameter to be estimated is not sufficient to ensure the degree of accuracy required by the specific application.

In the application developed, this performance increase allows to obtain accurate estimates of the properties of atmospheric and vacuum residues starting from the NMR spectrum of the oil, and in this substantially differs from all chemometric applications available to date in the scientific and patent literature. The proposed method can be used in principle with any fraction of the oil, even if, in the rest of the description, by way of illustration and not of limitation, reference will be made solely to the residues.

### Characterisation of oils and residues

The set of samples, collected over three years, consists of 70 oils from 18 different geographical areas, and presents a wide range of composition and chemical-physical properties. Table 1 shows maximum and minimum values of chemical and physical parameters of the analyzed oils and residues, analytically determined.

**Table 1**

| | | Oil | | Atmospheric residue | | Vacuum residue | |
|---|---|---|---|---|---|---|---|
| | U.M. | Min | Max | Min | Max | Min | Max |
| Sulfur | %p | 0,02 | 4,22 | 0,04 | 5,98 | 0,06 | 7,37 |
| Asphaltenes | %p | 0,02 | 8,57 | 0,05 | 13,47 | 0,08 | 24,07 |
| MCRT | %p | 0,41 | 12,53 | 0,97 | 18,57 | 2,93 | 30,14 |
| Ni | ppm | 0,40 | 58 | 0,91 | 104 | 2,61 | 219 |
| V | ppm | 0,14 | 106 | 0,34 | 187 | 0,80 | 382 |
| Fe | ppm | 0,40 | 18 | 0,57 | 30 | 1,51 | 65 |
| Acidity | mg_{KOH}/g | 0,02 | 2,23 | 0,04 | 2,73 | - | - |
| VBN @ 50°C | - | - | - | 29,10 | 45,28 | 35,35 | 54,77 |

The properties of the oils were determined by means of standard analytical methods ASTM. To obtain the residue, the samples were subjected to atmospheric distillation (atmospheric residue AR) and vacuum distillation (vacuum residue VR). The residues were then in turn characterised by means of standard analytical methods ASTM.

### NMR spectroscopy

The proton spectra (¹H NMR) used for the development of the method were obtained with a Varian Unity INOVA 300 spectrometer (Varian Inc., Palo Alto, CA (USA)) that operates at a frequency of 299,905 MHz.

A small part of the sample, well homogenized, was added with a few drops of tetramethylsilane (TMS), used as a reference for the resetting of the chemical shift. The sample was placed in a test tube, introduced in turn within a coaxial tube containing the deuterated solvent. In this way it was possible to obtain the spectra of the pure oils, without withdrawing the lock function performed by deuterium.

The acquisition was made with a spectral window of 4382Hz, equal to 14,6ppm, and with magnetization pulse of 90° (π/2) of the duration of 8,1µs and a recovery time between two successive transients of 30s. The spectra were obtained at a temperature of 25°C. The sequence is completed by the setting of a time of acquisition of 1,5s. The number of transients used for the acquisition of spectra with a good signal/noise ratio is 128.

After acquisition, all spectra have been subjected to the same processing, using a suitable software: timing, correction of the base line and zeroing of the scale in ppm with respect to TMS standard. Subsequently, each spectrum, in the range 0,22-9,00ppm, excluding the region 4,20-5,90ppm, which contains the water signal that would otherwise interfere with the analysis, was divided into regions with an amplitude of 0,04 ppm, the integrals of which were calculated; finally, the sum of the integrals was normalized.

### Multivariate analysis

In order to extract quantitative information of the residues, a multivariate calibration was performed. A multivariate calibration refers to the construction of a mathematical expression that binds a property, or a chemical physical parameter, to the experimental set of data, for example the absorbance as a function of wavelength in a set of IR spectra, or the intensity as a function of the chemichal shift in the case of NMR data. One of the most common methods of constructing these mathematical expressions is the partial least squares PLS regression. The PLS regression is a well-known multivariate analysis method, used to find the fundamental relationships between two data matrixs (X and Y). This method relates the dependent variable y with an independent set of variables X, through the reduction of the matrix in its main components and the use of the latter for the regression with the variable y. The PLS simultaneously models both matrixs X and Y to find the latent variables in X that best predict the latent variables in Y. The PLS regression is particularly suited to the NMR spectral data, that is, matrixs X having more variables that observable. In the specific case of the present invention,the PLS regression was implemented using the well-known PLS-1 algorithm.

On this basis, the 178 areas obtained by the ¹H NMR spectra constitute the elements of the matrix X (70 x 178) of spectral data, and the analytical data of the residues form the matrix Y of the chemical-physical properties.

The chemometric models for predicting properties of the residue from ¹H NMR spectra of the oils have been developed using a unique set for the calibration and validation, the model performance has been evaluated with the known cross-validation method with the exception of an object at a time, ie, with a leave-one-out cross-validation (CVloo). The performance of the models were evaluated in terms of: number of factors used, standard error in cross validation (SECV). The SECV is the standard deviation of the differences between the analytically measured values and those estimated from NMR models.

The models were developed using the software The Unscrambler X® 10.2 (CAMO Software AS, Computer Aided Modeling, Norway).

### PLS models calibration

The calibration process of PLS models is divided into two parts, one which is more properly computational and one which is analytical. The steps characterising the first stage can be summarized as follows:
I. selecting a set of oils;
II. obtaining the spectra of said oils through the ¹H NMR technique;
III. preparing atmospheric and vacuum residues by distillation of said oils;
IV. determining a set of chemical-physical properties of said oils by means of standard analytical methods;
V. determining a set of chemical-physical properties of said atmospheric and vacuum residues by means of standard analytical methods.

During the second step, the prediction models were constructed starting from the matrix Y of the chemical-physical properties of residues and the matrix Z, formed by the matrix X of the ¹H NMR spectra of the oils and the chemical-physical properties of the same oils. The matrix Z is called "expanded matrix".

The step of calibration of the PLS models of the atmospheric residue can be thus described:
VI. producing a set of "expanded spectra", obtained by the concatenation of the data of chemical-physical properties of step IV, with the spectra obtained in step II;
VII. regression, using the PLS method, of the set of "expanded spectra" obtained in step VI with the chemical-physical properties of the atmospheric residues of step V.

Similarly, the models of the vacuum residue were constructed by means of:
VIII. producing a set of "expanded spectra", obtained by the concatenation of the data of chemical-physical properties of the atmospheric residues of step V, with the spectra obtained in step II;
IX. regression, using the PLS method, of the set of "expanded spectra" obtained in step VIII with the chemical-physical properties of the vacuum residues of step V.

### Results and discussion

Models were developed for the characteristic properties of the residues: acidity (expressed as total acid numer or TAN), asphaltenes, MCRT carbon residue, the content of sulfur and metals (Ni, V, Fe) of the atmospheric residue were correlated using the ¹H NMR spectrum of the oil, from time to time expanded with the analogous properties of the oil; similarly, the viscosity blending number VBN, asphaltenes, MCRT carbon residue, the content of sulfur and metals (Ni, V, Fe) of the vacuum residue, were correlated using the spectrum ¹H NMR of the oil, expanded this time with the properties of the atmospheric residue. The performance of the proposed method were compared with those of the NMR models without making use of additional properties. To be able to assess with the same metric both models with a few samples and those with a greater number of samples, the validation in full cross validation was chosen. The performance of each model was evaluated by comparing the SECV, while the significance of the difference between the results obtained was evaluated using the F test (α=0,05). The data are shown in Tables 2 and 3, respectively, with respect to atmospheric residues (Table 2) and to vacuum residues (Table 3).

**Table 2**

| | | Sulfur_{AR} %p | Asphaltenes_{AR} %p | MCRT_{AR} %p | Ni_{AR} ppm | V_{AR} pp m | Fe_{AR} ppm | TAN_{AR} mg_{KOH}/g |
|---|---|---|---|---|---|---|---|---|
| Reproducibility of the analytical method in the field of measurement | Min | 0,005 | 0,01 | 0,19 | 1,0 | 1,0 | 0,3 | 0,15 |
| | Max | 0,29 | 2,7 | 1,0 | 30 | 80 | 13 | 0,53 |
| Input: Matrix X | SECV | 0,37 | 1,79 | 1,10 | 14 | 31 | 4,7 | 0,34 |
| Input: Matrix Z | SECV | 0,15 | 0,45 | 0,40 | 2,9 | 4,2 | 1,5 | 0,22 |

**Table 3**

| | | Sulfur_{VR} %p | Asphaltenes_{VR} %p | MCRT_{VR} %p | Ni_{VR} ppm | V_{VR} ppm | Fe_{VR} ppm | VBN_{VR} @ 50 °C |
|---|---|---|---|---|---|---|---|---|
| Reproducibility of the analytical method in the field of measurement | Min | 0,01 | 0,02 | 0,28 | 2,3 | 1,3 | 0,8 | - |
| | Max | 0,34 | 4,81 | 1,55 | 52 | 150 | 26 | - |
| Input: Matrix X | SECV | 0,54 | 3,6 | 3,18 | 42 | 71 | 10 | 1,01 |
| Input: Matrix Z | SECV | 0,19 | 1,25 | 1,11 | 7 | 18 | 4 | 0,72 |

In addition, attached figures reports the diagrams relating to certain parameters of the residue characterisation, measured and predicted from the PLS regression, both in the case derived from the matrix X of the ¹H NMR spectra of oil analytically determined both in the case derived from the matrix Z or expanded matrix, which shows the data of the so-called "expanded spectrum".

In particular, by way of example, for the AR cases have been reported for sulfur (Figures 1a, 1b), asphaltenes (Figures 2a, 2b), a metal (vanadium) (Figures 3a, 3b) and acid number TAN (Figures 4a, 4b). These properties are related in a different way with the spectrum and vary in terms of their distribution in the oil.

The performance of the prediction models were evaluated using the F-test: the variations were significant at 95% confidence. In the case of the TAN, the improvement is significant at 90% confidence.

As well as for the prediction models for the AR properties, the models for the VR properties have been developed using the matrix Z of the "expanded spectra", obtained this time by combining the matrix of ¹H NMR spectra of the oil with a column with the corresponding AR properties. In Figures 5-8 are graphically represented sulfur (Figures 5a and 5b), MCRT (Figures 6a and 6b), nickel (Figures 7a and 7b) and VBN (Figures 8a and 8b).

In general, the results of the prediction method according to the present invention are well represented in the graphs of regressions. For all the parameters, both of the AR and of the VR, it is observed a substantial improvement of performance by switching from the models that use only the spectra to those in which an analytical property is added: the SECV values decrease significantly, in some cases more than one half, for example, for the prediction models of the content of asphaltenes, MCRT and metals. The errors measured in the phase of experimental verification before the addition of the chemical-physical variable to the spectrum, confirm the results reported in the literature and the difficulty to characterize the residues starting from the spectrum of the oil. In contrast, the prediction method of chemical-physical properties of a fraction of a oil distillation according to the present invention provides more accurate results, not only with respect to the methods which make use of the spectra of the oils, but also with respect to the prediction methods developed using the spectra of the residues [Nielsen K.E., Dittmer J., Malmendal A., and Nielsen N.C., Energy & Fuels 2008, 22]. With the prediction method of the chemical-physical properties of a distillation fraction of a oil according to the present invention the current limits of the applications that use the IR and NMR spectroscopy to characterize the oils residues are overcome, without having to make the distillation.

The applications of the prediction method of chemical-physical properties of a distillation fraction of a oil according to the present invention may be different. In order to estimate the properties of the atmospheric residue it is sufficient to apply the prediction method of chemical-physical properties of a distillation fraction of a oil according to the present invention as such. In this case, for the analysis of an unknown sample, a small portion of the oil sample is taken for the acquisition of the proton spectrum while another portion is used for the determination of the required parameters (eg. asphaltenes, MCRT, TAN, sulfur, nickel, vanadium and iron). The spectrum is processed in the manner described in the experimental part and concatenated with the chemical-physical properties previously determined in the oil. The values of the properties of atmospheric residue are estimated by applying the prediction model according to the present invention to the expanded spectrum of the unknown sample.

If it is needed to estimate the properties of the vacuum residue, it would anyway be needed to make, according to a first embodiment of the present invention, at least the atmospheric distillation, to determine the properties of the atmospheric residue to be concatenated to the spectrum of the oil. In this way, the time required to perform the vacuum distillation and the analysis of the VR residue would be saved anyway. One of the advantages that would anyway derive from the application of this method, is definitely the saving of time, especially for those parameters for which, apart from distillation, the analytical determination requires rather long times (eg the MCRT carbon residue: one working day compared to a couple of hours required for the application of the proposed method).

Alternatively , according to a second embodiment of the present invention, the method for the VR can be used without the need to perform the analysis of the AR residue, concatenating to the spectrum of the oil the values estimated with the new method, instead of the analytically determined properties of the AR residue. Then, using the predicted properties, it is possible to obtain, without having to perform any distillation, both the estimates of the AR properties and those of VR properties.

### Conclusions

In conclusion, the method of prediction of chemical-physical properties of a distillation fraction of an oil according to the present invention provides for the use of PLS models on the expanded spectra, created by combining the NMR spectra with information of physical chemical character. It represents a valuable alternative to laboratory methods usually used to estimate the properties of oil residues, offering the huge advantage of avoiding the distillation of oil to its fractions before they can be characterised, with considerable savings in terms of time and money.

The correlations developed for some of the most important parameters used for the characterisation (sulfur, asphaltenes, MCRT, metals and viscosity), illustrate, both in the case of the atmospheric residues and in that of the vacuum residues, that adding a parameter to the spectrum of the oil, in the formulation of the calibration model, invariably results in a significant performance increase, which results in more robust and accurate models. By exploiting the accuracy of the method of the expanded spectra, it is possible to develop applications like the analysis of the residues starting from the spectra of oil that, so far, given the level of accuracy of traditional chemometric applications, were not possible.

Although in the description reference is made to the experimental verification only in the case of the specific application to atmospheric and/or vacuum residues of an oil, in principle the method of prediction according to the present invention is applicable to any complex mixture divisible into sub-samples, the percentage content of which is worthy of note and the properties of which are reflected on the starting mixture; making use of other spectroscopic techniques; using different algorithms; using chemical-physical properties for the creation of the "expanded spectra" which are different from those that it is desired to predict.

The present invention has been described for illustrative but not limitative purposes, according to its preferred embodiments, but it is to be understood that variations and/or modifications can be made by those skilled in the art without departing from the relevant scope of protection, as defined by the appended claims.

## Claims

1. Prediction method of chemical-physical properties of a fraction of an atmospheric distillation of an oil, which comprises the following steps:
- withdrawing a first sample of said oil and obtaining its 1H NMR spectrum;
- withdrawing a second sample of said oil and analytically determining, according to standard analytical methods, the same chemical-physical properties for which it is desired to make a prediction with respect to said fraction of atmospheric distillation;
- building an "expanded spectrum" by concatenating the 1H NMR spectrum of said first sample of said oil with the chemical and physical properties determined on said second sample of said oil;
- obtaining the desired property of said distillation fraction inputting the data obtained from the preceding steps in a mathematical expression, representative of a prediction model, previously obtained by:
I. selecting a set of oils similar to those on which the analysis will have to be carried out;
II. obtaining the spectra of said oils through the 1H NMR technique;
III. preparing atmospheric residues by distillation of said oils;
IV. determining a set of chemical-physical properties of said oils by means of standard analytical methods;
V. determining a set of chemical-physical properties of said atmospheric residues by means of standard analytical methods;
and subsequent building and calibrating of the prediction model through the following steps:
VI. producing a set of "expanded spectra", obtained by the concatenation of the data of chemical-physical properties of step IV, in the case of determination of chemical-physical properties of a fraction of atmospheric distillation with the spectra obtained in step II;
VII. making a regression, with a method of multivariate calibration, of the set of "expanded spectra" obtained in step VI with the chemical-physical properties of the atmospheric residues of step V.

2. Prediction method of chemical-physical properties of a fraction of vacuum distillation of an oil, which comprises the following steps:
- withdrawing a first sample of said oil and obtaining its 1H NMR spectrum;
- withdrawing a second sample of said oil and subjecting it to atmospheric distillation to obtain the residue and analytically determining, according to standard analytical methods, the same chemical-physical properties for which it is desired to make a prediction with respect to said fraction of vacuum distillation;
- building a "expanded spectrum" by concatenating the 1H NMR spectrum of said first sample of said oil with the chemical-physical properties determinated on said sample of the residue of said atmospheric distillation;
- obtaining the desired property of said fraction of vacuum distillation inputting the data obtained from the preceding steps in a mathematical expression, representative of a prediction model, previously obtained by:
I. selecting a set of oils similar to those on which the analysis will have to be carried out;
II. obtaining the spectra of said oils through the 1H NMR technique;
III. preparing atmospheric and vacuum residues by distillation of said oils;
IV. determining a set of chemical-physical properties of said oils by means of standard analytical methods;
V. determining a set of chemical-physical properties of said atmospheric and vacuum residues by means of standard analytical methods;
and subsequent building and calibrating of the prediction model through the following steps:
VI. producing a set of "expanded spectra", obtained by the concatenation of the data of chemical-physical properties of the atmospheric residues of step V with the spectra obtained in step II;
VII. making a regression, with a method of multivariate calibration, of the set of "expanded spectra" obtained in step VI with the chemical-physical properties of the atmospheric and vacuum residues of step V.

3. Prediction method of chemical-physical properties of a fraction of vacuum distillation of an oil, which comprises the following steps:
- withdrawing a first sample of said oil and obtaining its 1H NMR spectrum;
- withdrawing a second sample of said oil and determining, according to an application of the prediction method of claim 1, the chemical-physical properties of an atmospheric residue of which it is desired to make a prediction regarding said fraction of vacuum distillation;
- building a "expanded spectrum" by concatenating the 1H NMR spectrum of said first sample of said oil with the chemical-physical properties of a residue of atmospheric distillation of said oil sample;
- obtaining the desired property of said fraction of vacuum distillation inputting the data obtained from the preceding steps in a mathematical expression, representative of a prediction model, previously obtained by:
I. selecting a set of oils similar to those on which the analysis will have to be carried out;
II. obtaining the spectra of said oils through the 1H NMR technique;
III. preparing atmospheric and vacuum residues by distillation of said oils;
IV. determining a set of chemical-physical properties of said oils by means of standard analytical methods;
V. determining a set of chemical-physical properties of said atmospheric and vacuum residues by means of standard analytical methods;
and subsequent building and calibrating of the prediction model through the following steps
VI. producing a set of "expanded spectra", obtained by the concatenation of the data of chemical-physical properties of the atmospheric residues of step V with the spectra obtained in step II;
VII. making a regression, with a method of multivariate calibration, of the set of "expanded spectra" obtained in step VI with the chemical-physical properties of the atmospheric and/or vacuum residues of step V.

4. Prediction method of chemical-physical properties of a fraction of atmospheric and/or vacuum distillation of a oil according to any one of the preceding claims, **characterised in that** the spectra are concatenated in a plurality of chemical-physical properties of said oil or said fraction of atmospheric distillation.

5. Prediction method of chemical-physical properties of a fraction of atmospheric and/or vacuum distillation of a oil according to any one of the preceding claims, **characterised in that** the spectra are concatenated to a chemical-physical property of said oil or said fraction of atmospheric distillation different from that which is to be determined.

## Patentansprüche

1. Vorhersageverfahren für die chemisch-physikalischen Eigenschaften einer Fraktion einer atmosphärischen Destillation eines Öls, das die folgenden Schritte umfasst:
- Entnehmen einer ersten Probe des Öls und Erhalten seines 1H-NMR-Spektrums;
- Entnehmen einer zweiten Probe des Öls und analytisches Bestimmen der gleichen chemisch-physikalischen Eigenschaften, für die eine Vorhersage in Bezug auf die Fraktion der atmosphärischen Destillation erfolgen soll, nach üblichen analytischen Methoden;
- Aufbau eines "erweiterten Spektrums" durch Verketten des 1H-NMR-Spektrums der ersten Probe des Öls mit den chemischen und physikalischen Eigenschaften, die an der zweiten Probe des Öls bestimmt werden;
- Erhalten der gewünschten Eigenschaft der Destillationsfraktion unter Eingabe der aus den vorhergehenden Schritten erhaltenen Daten in einem mathematischen Ausdruck, der für ein Vorhersagemodell repräsentativ ist, das zuvor erhalten wurde durch:
I. Auswahl eines Satzes von Ölen, ähnlich denen, an denen die Analyse durchgeführt werden muss;
II. Erhalten der Spektren der Öle durch die 1H-NMR-Technik;
III. Herstellen von atmosphärischen Rückständen durch Destillation dieser Öle;
IV. Bestimmen eines Satzes chemisch-physikalischer Eigenschaften der Öle mit Hilfe von Standardanalysemethoden;
V. Bestimmen eines Satzes chemisch-physikalischer Eigenschaften der atmosphärischen Rückstände mit Hilfe von Standardanalysemethoden;
und anschließendes Erstellen und Kalibrieren des Vorhersagemodells durch die folgenden Schritte:
VI. Erzeugen eines Satzes von "erweiterten Spektren", erhalten durch die Verkettung der Daten der chemisch-physikalischen Eigenschaften von Schritt IV, im Falle der Bestimmung der chemisch-physikalischen Eigenschaften einer Fraktion der atmosphärischen Destillation mit den in Schritt II erhaltenen Spektren;
VII. Durchführen einer Regression mit einem Verfahren zur multivariaten Kalibrierung des in Schritt VI erhaltenen Satzes von "erweiterten Spektren" mit den chemisch-physikalischen Eigenschaften der atmosphärischen Rückstände von Schritt V.

2. Vorhersageverfahren für die chemisch-physikalischen Eigenschaften einer Fraktion der Vakuumdestillation eines Öls, das die folgenden Schritte umfasst:
- Entnehmen einer ersten Probe des Öls und Erhalten seines 1H-NMR-Spektrums;
- Entnehmen einer zweiten Probe des Öls und Unterwerfen desselben einer atmosphärischen Destillation, um den Rückstand zu erhalten, und analytisches Bestimmen der gleichen chemisch-physikalischen Eigenschaften, für die eine Vorhersage in Bezug auf den Anteil der Vakuumdestillation erfolgen soll, nach üblichen analytischen Methoden;
- Aufbau eines "erweiterten Spektrums" durch Verketten des 1H-NMR-Spektrums der ersten Probe des Öls mit den chemisch-physikalischen Eigenschaften, die an der Probe des Rückstands der atmosphärischen Destillation bestimmt werden;
- Erhalten der gewünschten Eigenschaft der Fraktion der Vakuumdestillation unter Eingabe der aus den vorhergehenden Schritten erhaltenen Daten in einen mathematischen Ausdruck, der für ein Vorhersagemodell repräsentativ ist, das zuvor erhalten wurde durch:
I. Auswahl eines Satzes von Ölen, ähnlich denen, an denen die Analyse durchgeführt werden muss;
II. Erhalten der Spektren der Öle durch die 1H-NMR-Technik;
III. Herstellen von atmosphärischen und Vakuumrückständen durch Destillation dieser Öle;
IV. Bestimmen eines Satzes chemisch-physikalischer Eigenschaften der Öle mit Hilfe von Standardanalysemethoden;
V. Bestimmen eines Satzes chemisch-physikalischer Eigenschaften der atmosphärischen und Vakuumrückstände mit Hilfe von Standardanalysemethoden;
und anschließendes Erstellen und Kalibrieren des Vorhersagemodells durch die folgenden Schritte:
VI. Erzeugen eines Satzes von "erweiterten Spektren", erhalten durch die Verkettung der Daten der chemisch-physikalischen Eigenschaften der atmosphärischen Rückstände von Schritt V mit den in Schritt II erhaltenen Spektren;
VII. Durchführen einer Regression mit einem Verfahren zur multivariaten Kalibrierung des in Schritt VI erhaltenen Satzes von "erweiterten Spektren" mit den chemisch-physikalischen Eigenschaften der atmosphärischen und Vakuumrückstände von Schritt V.

3. Vorhersageverfahren für die chemisch-physikalischen Eigenschaften einer Fraktion der Vakuumdestillation eines Öls, das die folgenden Schritte umfasst:
- Entnehmen einer ersten Probe des Öls und Erhalten seines 1H-NMR-Spektrums;
- Entnehmen einer zweiten Probe des Öls und Bestimmen der chemisch-physikalischen Eigenschaften eines atmosphärischen Rückstands, von dem eine Vorhersage über den Anteil der Vakuumdestillation gemacht werden soll, nach einer Anwendung des Vorhersageverfahrens nach Anspruch 1;
- Aufbau eines "erweiterten Spektrums" durch Verketten des 1H-NMR-Spektrums der ersten Probe des Öls mit den chemisch-physikalischen Eigenschaften eines Rückstands der atmosphärischen Destillation der Ölprobe;
- Erhalten der gewünschten Eigenschaft der Fraktion der Vakuumdestillation unter Eingabe der aus den vorhergehenden Schritten erhaltenen Daten in einen mathematischen Ausdruck, der für ein Vorhersagemodell repräsentativ ist, das zuvor erhalten wurde durch:
I. Auswahl eines Satzes von Ölen, ähnlich denen, an denen die Analyse durchgeführt werden muss;
II. Erhalten der Spektren der Öle durch die 1H-NMR-Technik;
III. Herstellen von atmosphärischen und Vakuumrückständen durch Destillation dieser Öle;
IV. Bestimmen eines Satzes chemisch-physikalischer Eigenschaften der Öle mit Hilfe von Standardanalysemethoden;
V. Bestimmen eines Satzes chemisch-physikalischer Eigenschaften der atmosphärischen und Vakuumrückständen mit Hilfe von Standardanalysemethoden;
und anschließendes Erstellen und Kalibrieren des Vorhersagemodells durch die folgenden Schritte
VI. Erzeugen eines Satzes von "erweiterten Spektren", erhalten durch die Verkettung der Daten der chemisch-physikalischen Eigenschaften der atmosphärischen Rückstände von Schritt V mit den in Schritt II erhaltenen Spektren;
VII. Durchführen einer Regression mit einem Verfahren zur multivariaten Kalibrierung des in Schritt VI erhaltenen Satzes von "erweiterten Spektren" mit den chemisch-physikalischen Eigenschaften der atmosphärischen und/oder Vakuumrückstände von Schritt V.

4. Vorhersageverfahren für die chemisch-physikalischen Eigenschaften einer Fraktion der atmosphärischen und/oder Vakuumdestillation eines Öls nach einem der vorstehenden Ansprüche, **dadurch gekennzeichnet, dass** die Spektren in einer Vielzahl von chemisch-physikalischen Eigenschaften des Öls oder der Fraktion der atmosphärischen Destillation verkettet sind.

5. Vorhersageverfahren für die chemisch-physikalischen Eigenschaften einer Fraktion der atmosphärischen und/oder Vakuumdestillation eines Öls nach einem der vorstehenden Ansprüche, **dadurch gekennzeichnet, dass** die Spektren mit einer chemisch-physikalischen Eigenschaft des Öls oder der Fraktion der atmosphärischen Destillation verkettet sind, die sich von der zu bestimmenden unterscheidet.

## Revendications

1. Procédé de prédiction de propriétés physicochimiques d'une fraction d'une distillation atmosphérique d'une huile, qui comprend les étapes suivantes consistant à :
- retirer un premier échantillon de ladite huile et obtenir son spectre RMN 1H ;
- retirer un second échantillon de ladite huile et déterminer analytiquement, selon des procédés analytiques standard, les mêmes propriétés physicochimiques pour lesquelles on souhaite faire une prédiction par rapport à ladite fraction de distillation atmosphérique ;
- élaborer un « spectre expansé » par concaténation du spectre RMN 1H dudit premier échantillon de ladite huile avec les propriétés physiques et chimiques déterminées sur ledit second échantillon de ladite huile ;
- obtenir la propriété souhaitée de ladite fraction de distillation en saisissant les données obtenues dans les étapes précédentes dans une expression mathématique représentative d'un modèle de prédiction, obtenu antérieurement par :
I. sélection d'un ensemble d'huiles similaires à celles sur lesquelles l'analyse devra être effectuée ;
II. obtention des spectres desdites huiles par la technique RMN 1H ;
III. préparation de résidus atmosphériques par distillation desdites huiles ;
IV. détermination d'un ensemble de propriétés physicochimiques desdites huiles au moyen de procédés analytiques standard ;
V. détermination d'un ensemble de propriétés physicochimiques desdits résidus atmosphériques au moyen de procédés analytiques standard ; et élaboration et calibrage ultérieurs du modèle de prédiction par les étapes suivantes consistant à :
VI. produire un ensemble de « spectres expansés » obtenus par la concaténation des données de propriétés physicochimiques de l'étape IV dans le cas de la détermination de propriétés physicochimiques d'une fraction de distillation atmosphérique avec les spectres obtenus à l'étape II ;
VII. effectuer une régression avec un procédé de calibrage multivariable de l'ensemble de « spectres expansés » obtenus à l'étape VI avec les propriétés physicochimiques des résidus atmosphérique de l'étape V.

2. Procédé de prédiction de propriétés physicochimiques d'une fraction de distillation sous vide d'une huile, qui comprend les étapes suivantes consistant à :
- retirer un premier échantillon de ladite huile et obtenir son spectre RMN 1H ;
- retirer un second échantillon de ladite huile et le soumettre à une distillation atmosphérique pour obtenir le résidu et déterminer analytiquement, selon des procédés analytiques standard, les mêmes propriétés physicochimiques pour lesquelles on souhaite faire une prédiction par rapport à ladite fraction de distillation sous vide ;
- élaborer un « spectre expansé » par concaténation du spectre RMN 1H dudit premier échantillon de ladite huile avec les propriétés physicochimiques déterminées sur ledit échantillon du résidu de ladite distillation atmosphérique ;
- obtenir la propriété souhaitée de ladite fraction de distillation sous vide en saisissant les données obtenues dans les étapes précédentes dans une expression mathématique représentative d'un modèle de prédiction, obtenu antérieurement par :
I. sélection d'un ensemble d'huiles similaires à celles sur lesquelles l'analyse devra être effectuée ;
II. obtention des spectres desdites huiles par la technique RMN 1H ;
III. préparation de résidus atmosphériques et sous vide par distillation desdites huiles ;
IV. détermination d'un ensemble de propriétés physicochimiques desdites huiles au moyen de procédés analytiques standard ;
V. détermination d'un ensemble de propriétés physicochimiques desdits résidus atmosphériques et sous vide au moyen de procédés analytiques standard ; et élaboration et calibrage ultérieurs du modèle de prédiction par les étapes suivantes consistant à :
VI. produire un ensemble de « spectres expansés » obtenus par la concaténation des données de propriétés physicochimiques des résidus atmosphériques de l'étape V avec les spectres obtenus à l'étape II ;
VII. effectuer une régression avec un procédé de calibrage multivariable de l'ensemble de « spectres expansés » obtenus à l'étape VI avec les propriétés physicochimiques des résidus atmosphériques et sous vide de l'étape V.

3. Procédé de prédiction de propriétés physicochimiques d'une fraction de distillation sous vide d'une huile, qui comprend les étapes suivantes consistant à :
- retirer un premier échantillon de ladite huile et obtenir son spectre RMN 1H ;
- retirer un second échantillon de ladite huile et déterminer selon une application du procédé de production de la revendication 1 les propriétés physicochimiques d'un résidu atmosphérique pour lequel on souhaite faire une prédiction concernant ladite fraction de distillation sous vide ;
- élaborer un « spectre expansé » par concaténation du spectre RMN 1H dudit premier échantillon de ladite huile avec les propriétés physicochimiques d'un résidu de distillation atmosphérique dudit échantillon d'huile ;
- obtenir la propriété souhaitée de ladite fraction de distillation sous vide en saisissant les données obtenues dans les étapes précédentes dans une expression mathématique représentative d'un modèle de prédiction, obtenu antérieurement par :
I. sélection d'un ensemble d'huiles similaires à celles sur lesquelles l'analyse devra être effectuée ;
II. obtention des spectres desdites huiles par la technique RMN 1H ;
III. préparation de résidus atmosphériques et sous vide par distillation desdites huiles ;
IV. détermination d'un ensemble de propriétés physicochimiques desdites huiles au moyen de procédés analytiques standard ;
V. détermination d'un ensemble de propriétés physicochimiques desdits résidus atmosphériques et sous vide au moyen de procédés analytiques standard ; et élaboration et calibrage ultérieurs du modèle de prédiction par les étapes suivantes consistant à :
VI. produire un ensemble de « spectres expansés » obtenus par la concaténation des données de propriétés physicochimiques des résidus atmosphériques de l'étape V avec les spectres obtenus à l'étape II ;
VII. effectuer une régression avec un procédé de calibrage multivariable de l'ensemble de « spectres expansés » obtenus à l'étape VI avec les propriétés physicochimiques des résidus atmosphériques et/ou sous vide de l'étape V.

4. Procédé de prédiction de propriétés physicochimiques d'une fraction de distillation atmosphérique et/ou sous vide d'une huile selon l'une quelconque des revendications précédentes, **caractérisé en ce que** les spectres sont concaténés dans une pluralité de propriétés physicochimiques de ladite huile ou de ladite fraction de distillation atmosphérique.

5. Procédé de prédiction de propriétés physicochimiques d'une fraction de distillation atmosphérique et/ou sous vide d'une huile selon l'une quelconque des revendications précédentes, **caractérisé en ce que** les spectres sont concaténés dans une propriété physicochimique de ladite huile ou de ladite fraction de distillation atmosphérique différente de celle qui doit être déterminée.
